# EUROPÄISCHE PATENTSCHRIFT

(11) **EP 3 716 842 B1**
(45) Veröffentlichungstag und Bekanntmachung des Hinweises auf die Patenterteilung: **07.02.2024**
(21) Anmeldenummer: 18826803.1
(22) Anmeldetag: 27.11.2018
(51) Int. Cl.: A61B 5/00, A61B 8/00, A61B 8/08, A61B 5/022

(54) **DRUCKMESSVORRICHTUNG ZUR DRUCKMESSUNG UND/ODER ELASTIZITÄTSMESSUNG EINER VENE ODER EINES ORGANS UND ZUR KOMBINATION MIT EINER ULTRASCHALLMESSEINHEIT, DRUCKMESSSYSTEM UND VERFAHREN**
PRESSURE MEASUREMENT DEVICE FOR MEASURING PRESSURE AND/OR FOR MEASURING ELASTICITY OF A VEIN OR AN ORGAN AND FOR COMBINATION WITH AN ULTRASONIC MEASUREMENT UNIT, PRESSURE MEASUREMENT SYSTEM, AND METHOD
DISPOSITIF DE MESURE DE PRESSION SERVANT À MESURER LA PRESSION ET/OU L'ÉLASTICITÉ D'UNE VEINE OU D'UN ORGANE ET DESTINÉ À ÊTRE COMBINÉ À UNE UNITÉ DE MESURE ULTRASONORE, SYSTÈME DE MESURE DE PRESSION ET PROCÉDÉ ASSOCIÉ

(30) Priorität: 28.11.2017 DE 102017221330
(43) Veröffentlichungstag der Anmeldung: 07.10.2020
(73) Patentinhaber: Compremium AG, 3074 Muri b. Bern (CH)
(72) Erfinder: BAUMANN, Ulrich A., 3110 Münsingen (CH)
(74) Vertreter: PPR AG
(86) Internationale Anmeldenummer: PCT/IB2018/059354
(87) Internationale Veröffentlichungsnummer: WO 2019/106535

(56) Entgegenhaltungen:
- EP-A1- 0 920 833
- EP-A2- 0 120 410
- DE-A1-102015 116 383
- US-A- 5 394 877
- US-A1- 2007 270 720
- BAUMANN U A ET AL: "Estimation of central venous pressure by ultrasound", RESUSCITATION, ELSEVIER, IE, Bd. 64, Nr. 2, 1. Februar 2005 (2005-02-01), Seiten 193-199, XP027603369, ISSN: 0300-9572 [gefunden am 2005-02-01]

## Beschreibung

Die Erfindung betrifft eine Druckmessvorrichtung nach dem Oberbegriff des Anspruchs 1 sowie ein Druckmesssystem zur Druckmessung und/oder Elastizitätsmessung von Venen, Organen oder Kompartimenten gemäss Anspruch 11 und eine Messeinrichtung zur Druckmessung und/oder Elastizitätsmessung einer Vene, eines Organs oder eines Kompartimenten nach Anspruch 12 sowie ein Verfahren zur Druckmessung und/oder Elastizitätsmessung einer Vene, eines Organs oder eines Kompartimenten gemäss Anspruch 13. Hierbei soll der Druck oder die Elastizität im Gewebe, vorzugsweise an einer Extremität eines Lebewesens, gemessen werden, wobei gleichzeitig das Gewebe mittels Ultraschall und dessen Reflexion am Gewebe auswertenden Verfahren beobachtet wird.

Unter dem Begriff "Kompartiment" wird in diesem Zusammenhang ein anatomisch definierter Raum verstanden, der gegenüber der Umgebung abgrenzbar ist. Neben Muskelkompartimenten stellt beispielsweise auch der Bauchraum ein Kompartiment dar.

Generell sind Ultraschalluntersuchungen mit Hilfe kommerziell erhältlicher in Ultraschallmesseinheiten, insbesondere Schallköpfen und bildgebenden Verfahren aus dem medizinischen Untersuchungsalltag bekannt. Dabei wird vom Schallkopf Ultraschall in das Gewebe eingestrahlt, dort reflektiert und die Laufzeitunterschiede werden durch bildgebende Verfahren zu einem Bild vom Inneren des zu untersuchenden Gewebes verarbeitet. Hierbei ist die Anwendung eines Druckes auf den Schallkopf zur Charakterisierung von druckbedingten Gewebe- und Gefässveränderungen gut etabliert. Insbesondere wird dies bei der Venenkompression zur Thrombosediagnostik angewendet. Im Falle dieser Untersuchungsverfahren hängt jedoch der ausgeübte Druck von der Erfahrung des Bedieners ab und ist von Untersucher zu Untersucher unterschiedlich. Im Zusammenhang mit der Darstellung von Gefässstrukturen und sich darin bewegenden Körperflüssigkeiten werden zur Bestimmung der Strömungsgeschwindigkeit insbesondere Dopplerverfahren eingesetzt, die eine Zusatzausrüstung zu kommerziellen Ultraschallgeräten bedingen.

Aus der EP 1 415 596 A1 ist eine Druckmessvorrichtung für Ultraschallmessvorrichtungen bekannt, die im Wesentlichen aus einem starren Behältnis besteht. An einer Flachseite ist eine steife Membran eingelassen, die der Ankopplung an den Ultraschallmesskopf dient. An der gegenüberliegenden Flachseite ist eine flexible Auflagemembran angebracht, welche den vom Gehäuse gebildeten Innenraum abschliesst. Der Innenraum der Druckmessvorrichtung ist mit einer ultraschalldurchlässigen Flüssigkeit gefüllt. Über eine Leitung kann der im Innenraum vorhandene Druck mittels einer externen Apparatur bestimmt werden.

Nachteilig an der bekannten Lösung ist, dass diese Druckmessvorrichtung zur Gewährleistung der hygienischen Anforderungen vorteilhaft als Einmalprodukt eingesetzt wird, aber Herstellungskosten aufweist, welche eine lediglich einmalige Verwendung derselben wirtschaftlich nicht rechtfertigen.

Aus der CH 707 046 B1 ist eine weitere Druckmessvorrichtung zur Druckmessung einer Vene oder eines Organs und zur Kombination mit einer Ultraschallmesseinheit bekannt, die sich in der Praxis sehr bewährt hat. Jedoch liegen auch bei dieser Druckmessvorrichtung die Herstellungskosten in einem Bereich, welcher eine lediglich einmalige Verwendung derselben wirtschaftlich nicht rechtfertigt. DE 10 2015 116383 A1 offenbart eine Druckmessvorrichtung zur Druckmessung und/oder Elastizitätsmessung einer Vene, eines Organs oder eines Kompartimentes und zur Kombination mit einer Ultraschallmesseinheit.

Aufgabe der vorliegenden Erfindung ist es somit, eine Druckmessvorrichtung, ein Druckmesssystem, eine Messeinrichtung sowie ein Verfahren zur Druckmessung und/oder Elastizitätsmessung von Venen, Organen oder Kompartimenten zu schaffen, welche kostengünstiger herstellbar als bisherige Lösungen sowie einfach und zuverlässig bedienbar sind.

Die Aufgabe wird für eine Druckmessvorrichtung gemäss den Merkmalen des Patentanspruches 1 und für ein Druckmesssystem gemäss den Merkmalen des Patentanspruches 11 sowie für eine Messeinrichtung gemäss den Merkmalen des Patentanspruchs 12 gelöst, während der Patentanspruch 13 eine Lösung für ein Verfahren zur Druckmessung und/oder Elastizitätsmessung einer Vene, eines Organs oder eines Kompartimentes angibt.

Gemäss der Erfindung ist ein als Foliendrucksensor ausgebildeter Drucksensor vorgesehen, wobei ein Zwischenraum zwischen den Folien des Foliendrucksensors mit einer ultraschalltransparenten sowie nicht-elektrolytisch aktiven Flüssigkeit befüllt ist.

Foliendrucksensoren der relevanten Art, wie z. B. ein Force Sensing Resistor (FSR) der Firma Interlinks Electronics, weisen eine Sandwichbauweise mit zwei Folien auf. Die erste Folie ist innenseitig elektrisch beschichtet bzw. bedruckt. Die andere Folie weist innenseitig ein elektrisches Kontaktgitter auf, das mittels Bedruckung oder Beschichtung aufgebracht wurde. Durch einen Abstandhalter sind die beiden Folien in einem Abstand zueinander gehalten und ein direkter Kontakt der beschichteten und/oder bedruckten Folienabschnitte wird verhindert. Der Abstandhalter ist derart ausgeformt, dass ein befüllbarer Zwischenraum ausgebildet ist. Der Abstand der Folien zueinander wird je nach Aufgabe ausgeführt und kann sehr geringgehalten werden, was eine flache Ausgestaltung des Foliendrucksensors ermöglicht. Bei Druck auf den Foliendrucksensor kommen die beschichteten und/oder bedruckten Seiten der Folien in Kontakt und bilden elektrische Brücken abhängig vom ausgeübten Druck. Dabei ändert sich der messbare elektrische Widerstand und die Grösse des ausgeübten Drucks lässt sich anhand desselben bestimmen.

Bei einer Elastizitätsmessung einer Vene, eines Organs oder eines Kompartementes wird ein Elastizitätsquotient ermittelt und mit einem Venendruck, einem Organdruck oder einem Kompartimentdruck korreliert.

Durch die Befüllung des Zwischenraums des Foliendrucksensors mit einer ultraschalltransparenten sowie nicht-elektrolytisch aktiven Flüssigkeit wird ein kommerzieller Foliendrucksensor ultraschalldurchlässig. Dadurch kann eine zuverlässige Druckmessung bei geringen Kosten der Druckmessvorrichtung gewährleistet werden.

In einer Ausführungsform wird der Zwischenraum während der Herstellung des Foliendrucksensors mit der ultraschalltransparenten sowie nicht-elektrolytisch aktiven Flüssigkeit befüllt.

Alternativ erfolgt die Befüllung nach der Herstellung des Foliendrucksensors durch repetitive Kompression in Immersion oder Vakuum. Eine weitere Möglichkeit stellt eine direkte Injektion der ultraschalltransparenten sowie nicht-elektrolytischen Flüssigkeit in den Zwischenraum dar.

Vorzugsweise sind die Folien des Foliendrucksensors zumindest bereichsweise aus einem ultraschalldurchlässigen Material gefertigt, womit die Ultraschall-Durchlässigkeit des Foliendrucksensors weiter verbessert wird.

Bevorzugterweise ist die ultraschalltransparente sowie nicht-elektrolytisch aktive Flüssigkeit eine Flüssigkeit aus der Gruppe der wässrigen Flüssigkeiten, Ultraschall-durchlässige Gele, synthetische Öle oder biologische Öle, welche eine gute Ultraschall-Durchlässigkeit des Foliendrucksensors gewährleistet. Als besonders bevorzugt haben sich biologische Öle erwiesen.

Vorzugsweise ist zumindest ein Druckübertragungselement vorgesehen, welches eine genaue Druckmessung auch auf elastischen Unterlagen gewährleistet, wie es die Haut bzw. das Gewebe darstellt. Bei elastischen Unterlagen kann sich eine Abflachung der Druckverlaufskurve einstellen, welche nicht dem effektiven Druckverlauf entspricht. Dies wird beispielsweise durch ein Saugphänomen zwischen den Abstandelementrändern und der elastischen Unterlage bewirkt. Mittels des zumindest einen Druckübertragungselements kann ein solches Saugphänomen vermieden und eine bessere Druckübertragung sichergestellt werden.

Das zumindest eine Druckübertragungselement ist vorteilhaft aus einem ultraschalldurchlässigen Material gefertigt, womit das zumindest eine Druckübertragungselement die Ultraschallmessung nicht oder nur eingeschränkt beeinflusst. Weiter vorteilhaft ist das zumindest eine Druckübertragungselement aus dem gleichen Material wie die Folie gefertigt, womit keine durch Materialunterschiede bedingte Einflüsse bei der Druckmessung und/oder der Elastizitätsmessung der Vene oder des Organs auftreten. Besonders vorteilhaft ist das zumindest eine Druckübertragungselement mit bzw. an der Folie ausgeformt, womit der Einfluss des zumindest eine Druckübertragungselements auf die Druckmessung und/oder der Elastizitätsmessung der Vene, des Organs oder des Kompartimentes zusätzlich reduziert wird.

Bevorzugterweise ist das zumindest eine Druckübertragungselement an einer mit der Ultraschallmesseinheit in Anlage bringbare Aussenseite des Foliendrucksensors oder der Druckmessvorrichtung angeordnet, womit eine optimale Druckübertragung durch die Ultraschallmesseinheit gewährleistet wird.

Vorteilhaft weist das zumindest eine Druckübertragungselement eine Höhenerstreckung auf, die zumindest dem Abstand der Folien des Foliendrucksensors zueinander entspricht, womit eine optimale Druckübertragung durch die Ultraschallmesseinheit gewährleistet wird.

Als besonders vorteilhaft für eine optimale Druckübertragung hat sich in Versuchen eine Höhenerstreckung erwiesen, die dem 5-fachen bis 20-fachen des Abstands der Folien des Foliendrucksensors zueinander entspricht.

Weiter vorteilhaft weist das zumindest eine Druckübertragungselement eine Ausdehnung auf, die etwa 30% bis 70% der entsprechenden Ausdehnung des Zwischenraums des Foliendrucksensors entspricht. womit eine optimale Druckübertragung durch die Ultraschallmesseinheit gewährleistet wird.

In Versuchen für eine vorteilhafte Druckübertragung hat sich eine Ausdehnung des zumindest einen Druckübertragungselements erweisen, die etwa 40% bis 60% der entsprechenden Ausdehnung des Zwischenraums des Foliendrucksensors entspricht.

In einer Alternative dazu ist das zumindest eine Druckübertragungselement bevorzugterweise an einer mit der Haut bzw. dem Gewebe in Anlage bringbare Aussenseite des Foliendrucksensors oder der Druckmessvorrichtung angeordnet, womit eine optimale Druckübertragung über die Druckmessvorrichtung durch die Ultraschallmesseinheit gewährleistet wird.

Dieses zumindest eine Druckübertragungselement weist vorteilhaft eine Ausdehnung auf, die etwa 30% bis 70% der entsprechenden Ausdehnung des Zwischenraums des Foliendrucksensors entspricht, womit eine optimale Druckübertragung über die Druckmessvorrichtung durch die Ultraschallmesseinheit gewährleistet wird.

Vorteilhaft weist das zumindest eine Druckübertragungselement eine Höhenerstreckung auf, die zumindest dem Abstand der Folien des Foliendrucksensors zueinander entspricht, womit eine optimale Druckübertragung durch die Ultraschallmesseinheit gewährleistet wird.

Als besonders vorteilhaft für eine optimale Druckübertragung hat sich in Versuchen eine Höhenerstreckung erwiesen, die dem 5-fachen bis 20-fachen des Abstands der Folien des Foliendrucksensors (252) zueinander entspricht.

In einer weiteren alternativen Ausführungsform ist sowohl an der mit der Ultraschallmesseinheit in Anlage bringbaren Aussenseite als auch an der mit der Haut bzw. dem Gewebe in Anlage bringbare Aussenseite des Foliendrucksensors oder der Druckmessvorrichtung jeweils ein Druckübertragungselement angeordnet, womit sich die Vorteile der einen Anordnung des Druckübertragungselements mit den Vorteilen der anderen Anordnung des Druckübertragungselements kombinieren lassen.

Bevorzugterweise ist ein Reservoir für die ultraschalltransparente sowie nicht-elektrolytisch aktive Flüssigkeit vorgesehen, wobei zwischen dem Reservoir und dem Zwischenraum des Foliendrucksensors eine Flüssigkeitsverbindung vorgesehen ist. Damit wird die Gebrauchstauglichkeit und die Lagerfähigkeit der Druckmessvorrichtung wesentlich verlängert, da die ultraschalltransparente sowie nicht-elektrolytisch aktive Flüssigkeit austreten, verdunsten oder eindicken kann. Aus messtechnischen Gründen ist eine Öffnung zum Zwischenraum des Foliendrucksensors vorteilhaft und sollte nicht verschlossen werden.

Vorteilhaft ist der Foliendrucksensor in dem Reservoir angeordnet, womit eine kompakte Ausgestaltung des Foliendrucksensors ermöglicht und ein einfacher Austausch der ultraschalltransparenten sowie nicht-elektrolytisch aktiven Flüssigkeit zwischen dem Foliendrucksensor und dem Reservoir gewährleistet ist.

Vorzugsweise ist eine Datenleitung, z. B. ein Verbindungskabel, zur Weitergabe der vom Foliendrucksensor ermittelten Werte vorgesehen, womit diese Werte einfach an eine Auswerteeinrichtung übermittelt werden können.

Alternativ ist ein Sender zur Weitergabe der vom Foliendrucksensor ermittelten Werte vorgesehen, wobei diese Werte einfach an eine Auswerteeinrichtung übermittelt werden können, ohne dass eine Datenleitung das Handling der Druckmessvorrichtung nicht einschränkt. Als Übermittlungsstandard wird eine Funkverbindung bevorzugt, beispielsweise ein Bluetooth^{®}- oder Zigbee^{®}-Verbindung.

Bevorzugterweise ist eine Halteeinrichtung für die Druckmessvorrichtung vorgesehen, womit die Druckmessvorrichtung bedarfsweise festlegbar ist und eine verbesserte Messung ermöglicht wird.

Vorteilhaft ist die Halteeinrichtung derart ausgebildet, dass diese auf einem Gewebe temporär festlegbar ist. Beispielsweise umfasst die Halteeinrichtung zumindest bereichsweise Klebeflächen, analog eines Pflasters, welche an dem Gewebe haften. In einer Alternative dazu umfasst die Halteeinrichtung Haltebänder, welche um ein Körperteil oder Körperabschnitt herumlegbar und fixierbar sind, z. B. über Klettverschlüsse.

Vorzugsweise ist der Foliendrucksensor zumindest bereichsweise in einem ultraschalldurchlässigen Einbettungsmaterial eingebettet, womit die erfindungsgemässe Druckmessvorrichtung eine gewisse Steifigkeit aufweist und eine längere Gebrauchstauglichkeit gewährleistet.

Vorzugsweise ist das Einbettungsmaterial ein ultraschalldurchlässiges Silikon, welches einfach verarbeitbar ist.

Vorteilhaft ist die Einbettung des Foliendrucksensors zumindest an der mit der Haut bzw. dem Gewebe in Anlage bringbare Aussenseite des Foliendrucksensors vorgesehen, womit eine ausreichende Steifigkeit der Druckmessvorrichtung für die meisten Anwendungen der erfindungsgemässen Druckmessvorrichtung gegeben ist.

In einer alternativen Ausführungsform ist der Foliendrucksensor vollständig in dem Einbettungsmaterial eingebettet, womit ein in der Handhabung einfach verwendbares Bauteil geschaffen wird, das sich auch in Vorrichtungen konstruktiv einfach integrieren lässt.

Ein erfindungsgemässes Druckmesssystem zur Druckmessung und/oder Elastizitätsmessung einer Vene oder eines Organs weist eine erfindungsgemässe Druckmessvorrichtung mit zumindest einem der vorgenannten Merkmale und eine Ultraschallmesseinheit auf, wobei die Druckmessvorrichtung und die Ultraschallmesseinheit miteinander zu einer Messeinheit gekoppelt sind. Dieses Drucksystem ist leicht transportabel und kann kompakt ausgebildet werden.

Vorteilhaft sind die Druckmessvorrichtung und die Ultraschallmesseinheit mechanisch zu einer Messeinheit koppelbar, was eine einfache Verbindung der beiden Bestandteile ermöglicht. Vorteilhaft ist die mechanische Koppelung lösbar ausgebildet, womit insbesondere die Druckmessvorrichtung bedarfsweise einfach austauschbar ist.

In einer Variante dazu sind die Druckmessvorrichtung und die Ultraschallmesseinheit zu einer Messeinheit verbunden.

Eine erfindungsgemässe Messeinrichtung zur Druckmessung und/oder Elastizitätsmessung einer Vene, eines Organs oder eines Kompartimentes weist wenigstens eine Verarbeitungseinheit und ein Druckmesssystem auf, das eine Ultraschallmesseinheit und eine zuvor beschriebene Druckmessvorrichtung aufweist. Die Messeinrichtung weist in der Handhabung eine Kombination der vorgenannten Vorteile auf. Durch die einfache Bedienbarkeit reduziert sich die Einarbeitungszeit des Bedienpersonals, bei Gewährleistung einer sicheren Anwendung.

Auf besonders vorteilhafte Weise lässt sich bei einer Weiterbildung des erfindungsgemässen Druckmesssystems so der Druck und/oder die Elastizität einer kollabierenden Vene bzw. der Verformungsgrad eines Organs oder eines Kompartimentes bestimmen. Ein Organ oder ein Kompartiment muss für die Druckmessung und/oder Elastizitätsmessung nicht vollständig im Ultraschall abgebildet werden, da die Druckmessung und/oder Elastizitätsmessung mit Gewährleistung von wiederholbaren und zuverlässigen Messergebnissen auch nur an einem Organteil oder an einem Ausschnitt oder Abschnitt eines Kompartiments ("Kompartimentsfenster") erfolgen kann. Dies trifft insbesondere dann zu, wenn der untersuchte Abschnitt für das Restorgan repräsentativ ist. Dadurch sind auch grosse Organe, wie z. B. eine Leber oder eine Muskelloge, messbar.

Besonders vorteilhaft lässt sich bei einer Weiterbildung des erfindungsgemässen Druckmesssystems der Zeitpunkt des Kollabierens der Vene automatisch, beispielsweise durch Computerauswertung der Ultraschallmesswerte, in Verbindung mit den Druckmesswerten bestimmen.

Besonders vorteilhaft lässt sich bei einer Weiterbildung des erfindungsgemässen Druckmesssystemsystems der Zeitpunkt des Venenkollabierens durch bildverarbeitende und/oder bildauswertende Verfahren bestimmen, welche Ultraschallbilder analysieren.

Besonders vorteilhaft lässt sich bei einem Verfahren zur Druckmessung und/oder Elastizitätsmessung einer Vene eines Organs oder eines Kompartimentes gemäss der Erfindung ein Druck und/oder eine Elastizität mit einer erfindungsgemässen Druckmessvorrichtung bestimmen, indem teilautomatisiert die kombinierte Ultraschallmesseinheit- und Druckmessvorrichtung auf ein Gewebe aufgedrückt wird, bis über eine Signalauswertung erkannt wird, dass eine Vene kollabiert oder ein bestimmter Verformungsgrad eines Organs oder Kompartimentes erreicht ist und dabei der entsprechende Druck in dem Foliendrucksensor gemessen wird.

Vorzugsweise wird eine Software zur Durchführung des bildverarbeitenden Verfahrens zur Verfügung gestellt und eingesetzt. Eine solche Software stellt für sich eine eigenständige Erfindung dar. Der kollabierte Zustand einer Vene wird dabei durch die Umrechnung von Rohdaten (Pixeln) in eine durchströmte Querschnittsfläche der Vene erkannt. Als Kriterium für das Kollabieren kann eine gegenüber dem Querschnitt der unbelasteten Vene beliebige Querschnittsreduktion definiert werden. Bei Organen wird demensprechend durch die Verschiebung von gewebebedingten Pixelanormalitäten (z. B. Blutgefässe, Dichteunterschiede, etc.) der Verformungsgrad des Organs oder des Organteils bestimmt. Wie bereits ausgeführt wurde, muss für eine Druckmessung und/oder Elastizitätsmessung mit der erfindungsgemässen Druckmessvorrichtung eines Organs dieses nicht vollständig im Ultraschall abgebildet werden.

In einer vorteilhaften Weiterbildung der Software ist das automatische Erkennen des Beginns des Messzyklus durch den Beginn des Deformierens der Vene bzw. durch den Beginn der Verringerung der durchströmten Querschnittsfläche definiert. Bei Organen oder Organteilen wird vorteilhaft der Zeitpunkt gewählt, bei dem sich Pixelanormalitäten zu verschieben beginnen.

In einer weiteren vorteilhaften Weiterbildung der Software ist das automatische Erkennen der Messqualität durch Bestimmen der Lage der beobachteten Vene bzw. des Organs oder des Organteils gegenüber der Messachse des Ultraschallmesskopfs bzw. Ultraschallwandlers und/oder des Foliendrucksensors der Druckmessvorrichtung definiert. Vorteilhaft wird dabei auch die Lage der Vene bzw. des Organs gegenüber benachbarten und insbesondere darunterliegenden Strukturen, wie Knochen oder dergleichen berücksichtigt.

In einer vorteilhaften Weiterbildung der Software erfolgt ein automatischer Entscheid über akzeptable oder nicht akzeptable Messung durch Berücksichtigung und Analyse aller vorgenannten Daten.

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung beschrieben sind.

Die Bezugszeichenliste ist wie auch der technische Inhalt der Patentansprüche und Figuren Bestandteil der Offenbarung. Die Figuren werden zusammenhängend und übergreifend beschrieben. Gleiche Bezugszeichen bedeuten gleiche Bauteile, Bezugszeichen mit unterschiedlichen Indices geben funktionsgleiche oder ähnliche Bauteile an.

Es zeigen dabei:
- Fig. 1: eine Messeinrichtung mit einem erfindungsgemässen Druckmesssystem in einer schematischen Darstellung,
- Fig. 2: ein Messvorgang mit der erfindungsgemässen Druckmessvorrichtung gem. Figur 1,
- Fig. 3: eine zweite Ausführungsform einer erfindungsgemässen Druckmessvorrichtung in einem ersten Zustand im Grundriss,
- Fig. 4: die Druckmessvorrichtung gem. Figur 3 in einem zweiten Zustand,
- Fig. 5: die Druckmessvorrichtung gem. Figur 3 in einem weiteren Zustand,
- Fig. 6: ein erfindungsgemässes Druckmesssystem in einer schematischen Darstellung,
- Fig. 7: eine erfindungsgemässe Druckmessvorrichtung mit einer Halteeinrichtung in einer schematischen Schnittdarstellung,
- Fig. 8: eine weitere Ausführungsform eines erfindungsgemässen Druckmesssystems in einer schematischen Darstellung,
- Fig. 9: eine weitere Ausführungsform einer erfindungsgemässen Druckmessvorrichtung im Grundriss, und
- Fig. 10: die erfindungsgemässe Druckmessvorrichtung gem. Figur 9 in einer Seitenansicht.

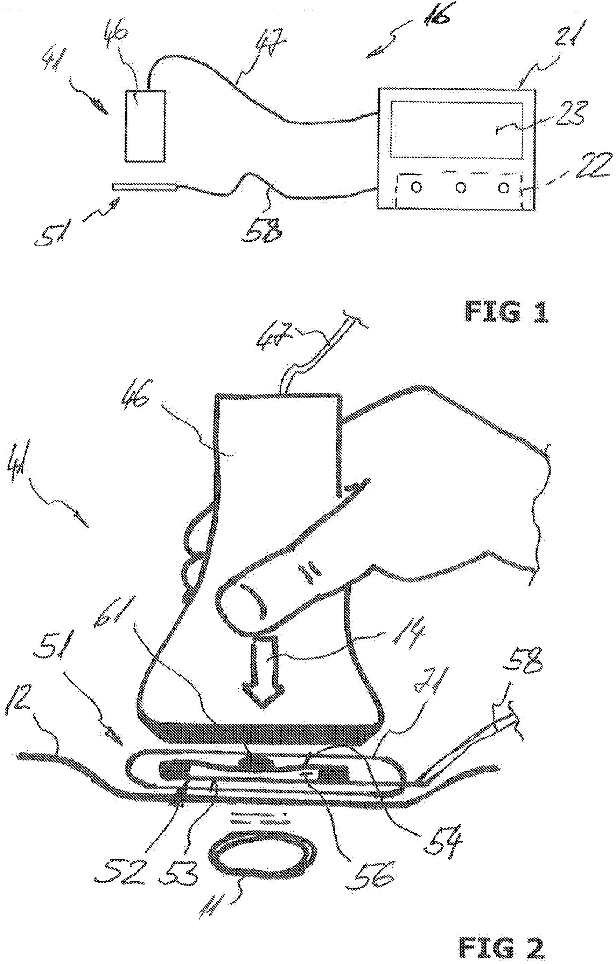

Die in Fig. 1 gezeigte Messeinrichtung 16 zur Druckmessung und/oder Elastizitätsmessung einer Vene, eines Organs oder eines Kompartimentes umfasst eine Verarbeitungseinheit 21 und ein Druckmesssystem 41, das eine Ultraschallmesseinheit 46 und eine Druckmessvorrichtung 51 aufweist.

Die Verarbeitungseinheit 21 beinhaltet eine Recheneinheit 22 zur Verarbeitung der von der Ultraschallmesseinheit 46 und von der Druckmessvorrichtung 51 erfassten Daten sowie eine Anzeigeeinheit 23, wie ein Display oder ein Touchscreen, zur Anzeige der verarbeiteten bzw. erfassten Daten.

Die von der Ultraschallmesseinheit 46 erfassten Daten werden über eine Datenleitung 47 von der Ultraschallmesseinheit 46 an die Verarbeitungseinheit 21 übermittelt. Die von der Druckmessvorrichtung 51 erfassten Daten werden über eine Datenleitung 58 von der Druckmessvorrichtung 51 an die Verarbeitungseinheit 21 übermittelt.

In der Fig. 2 ist das Druckmesssystem 41 zur Druckmessung einer Vene 11, eines Organs oder eines Kompartimentes in einer vergrösserten Darstellung gezeigt. Die Ultraschallmesseinheit 46 umfasst einen Ultraschallkopf, der Ultraschall aussendet und zurückreflektierenden Ultraschall erfasst.

Der Auflagedruck wird durch eine Kraft 14 auf den Ultraschallmesskopf an der Ultraschallmesseinheit 46 auf das Gewebe 12, wie zum Beispiel die Haut einer Person, beispielsweise an einem Arm, ausgeübt und aufgebracht. Der resultierende Druck bzw. die resultierende Elastizität kann während der Untersuchung quantitativ gemessen und dargestellt werden. Zu diesem Zweck ist die Druckmessvorrichtung 51 vorgesehen.

Die Druckmessvorrichtung 51 weist einen als Foliendrucksensor 52 ausgebildeten Drucksensor auf. Ein Zwischenraum 56 zwischen den Folien 53 und 54 des Foliendrucksensors 52 ist mit einer ultraschalltransparenten sowie nicht-elektrolytisch aktiven Flüssigkeit befüllt.

Die ultraschalltransparente sowie nicht-elektrolytisch aktive Flüssigkeit ist eine Flüssigkeit aus der Gruppe der wässrigen Flüssigkeiten, Ultraschall-durchlässige Gele, synthetische Öle oder biologische Öle. Als besonders bevorzugt haben sich biologische Öle erwiesen, wie beispielsweise Blaser Swisslube Typ 700-01.

Die Folien 53 und 54 des Foliendrucksensors 52 sind bereichsweise aus einem ultraschalldurchlässigen Material gefertigt. Beispielsweise sind die Folien aus Polytetrafluorethylen ausgeführt, abgekürzt PFTE und als Markenname mit der Bezeichnung Teflon von Dupont registriert.

Ein ultraschalldurchlässiges Druckübertragungselement 61 ist vorgesehen, welches an einer mit der Ultraschallmesseinheit 46 in Anlage bringbare Aussenseite der Folie 54 des Foliendrucksensors 52 angeordnet ist.

Der Foliendrucksensor 52 ist in einem Reservoir 71 für die ultraschalltransparente sowie nicht-elektrolytisch aktive Flüssigkeit angeordnet. Das Reservoir 71 dient der Aufnahme der aus dem Foliendrucksensor 52 austretenden ultraschalltransparenten sowie nicht-elektrolytisch aktiven Flüssigkeit und der Abgabe der in den Foliendrucksensor 52 zurückfliessenden ultraschalltransparenten sowie nicht-elektrolytisch aktiven Flüssigkeit.

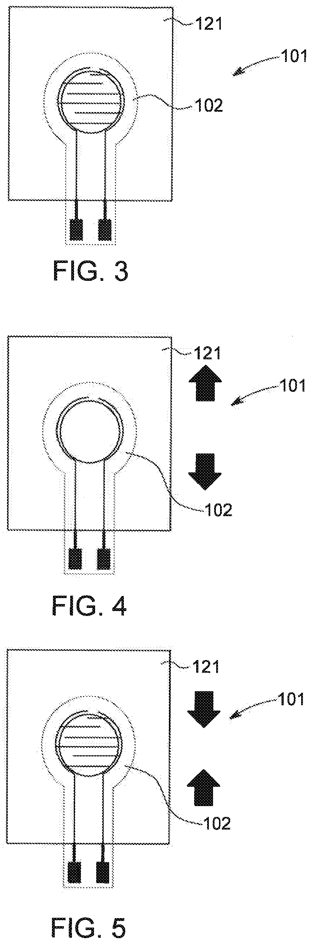

Die in den Figuren 3 bis 5 dargestellte Druckmessvorrichtung 101 weist ebenfalls einen Foliendrucksensor 102 als Drucksensor auf, der in einem Reservoir 121 für die ultraschalltransparente sowie nicht-elektrolytisch aktive Flüssigkeit angeordnet ist. Zwischen dem Reservoir 121 und dem Zwischenraum des Foliendrucksensors 102 ist eine Flüssigkeitsverbindung vorgesehen.

Figur 3 zeigt den Foliendrucksensor 102 ohne eine Messdruckbelastung. Der Zwischenraum des Foliendrucksensors 102 ist vollständig und das Reservoir 121 teilweise mit der ultraschalltransparenten sowie nicht-elektrolytisch aktiven Flüssigkeit befüllt.

Figur 4 zeigt den Foliendrucksensor 102 unter maximaler Messdruckbelastung. Die ultraschalltransparente sowie nicht-elektrolytisch aktive Flüssigkeit ist nahezu vollständig aus dem Zwischenraum des Foliendrucksensors 102 in das Reservoir 121 geflossen.

Figur 5 zeigt den Foliendrucksensor 102 nach der Messung ohne eine Messdruckbelastung. Die ultraschalltransparente sowie nicht-elektrolytisch aktive Flüssigkeit fliesst aus dem Reservoir 121 wieder in den Zwischenraum des Foliendrucksensors 102 zurück.

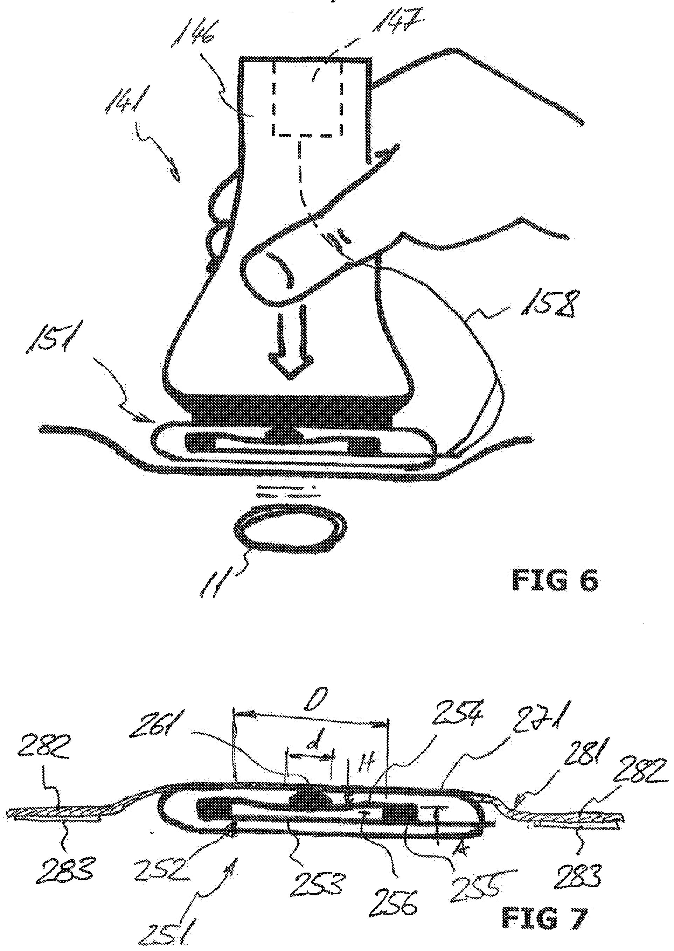

Bei dem in Fig. 6 dargestellten Druckmesssystem 141 zur Druckmessung einer Vene 11 oder eines Organs die Druckmessvorrichtung 151 und die Ultraschallmesseinheit 146 zu einer Messeinheit verbunden.

Die Druckmessvorrichtung 151 könnte auch mit der Ultraschallmesseinheit 146 zu einer Messeinheit gekoppelt sein, wobei die Kopplung vorteilhaft mechanisch und weiter vorteilhaft lösbar ausgebildet ist. Eine mechanische Kopplung ist für einen Fachmann einfach und sicher ausführbar. Mit einer lösbaren Kopplung lässt sich ein verwendeter Foliendrucksensor 151 einfach auswechseln.

An der Ultraschallmesseinheit 146 ist ein Sender 147 vorgesehen, so dass die Datenleitung durch eine Funkverbindung, welche aus einem Sender 147 und aus einem Empfänger besteht, ersetzt ist. Der Sender 147 ist zur Weitergabe der von der Ultraschallmesseinheit 146 sowie von dem Foliendrucksensor 151 erfassten Werte vorgesehen. Der Foliendrucksensor 151 ist in diesem Beispiel über eine Datenleitung 158 mit dem Sender 147 verbunden. In einer alternativen, hier nicht gezeigten Ausführung kann der Foliendrucksensor 151 einen Sender zur Übermittlung von Daten aufweisen.

Die Ausführung der Funkstrecke kann auf eine aus dem Stand der Technik bekannte Weise erfolgen. Sie kann unidirektional oder bidirektional sein. Gegebenenfalls bieten sich der Bluetooth^{®} oder Zigbee^{®} Standard als Grundlage der Funkverbindung an.

Weiterhin besteht die Möglichkeit, die vom Sender 147 abgegebenen Daten mehrfach mittels mehrerer Empfangsgeräte zu empfangen und in geeigneter Weise weiterzuverarbeiten. Beispielsweise können Druckmessreihen gespeichert werden, um systematische Messfehler zu ermitteln. Ferner können zu Schulungszwecken unterschiedliche Messdatensätze miteinander verglichen werden.

Bei der Druckmessvorrichtung 251 gemäss Fig. 7 ist der Foliendrucksensor 252 ebenfalls in einem Reservoir 271 angeordnet. Der Foliendrucksensor 252 weist eine Folie 253 und eine Folie 254 auf, die über einen Abstandhalter 255 zueinander beanstandet sind. Der Abstandhalter 255 sowie die Folien 253 und 254 umschliessen den mit einer ultraschalltransparenten sowie nicht-elektrolytisch aktiven Flüssigkeit befüllten Zwischenraum 256.

Die Folie 253 ist innenseitig, d. h. auf der der anderen Folie 254 zugewandten Seite, elektrisch bedruckt. Die andere Folie 254 weist innenseitig, d. h. auf der der ersten Folie 253 zugewandten Seite, ein elektrisches Kontaktgitter auf, das mittels Bedruckung aufgebracht wurde. Durch den Abstandhalter 255 sind die beiden Folien 253 und 254 in einem Abstand zueinander gehalten und ein direkter Kontakt der bedruckten Folienabschnitte ist verhindert. Bei Druck auf den Foliendrucksensor 251 kommen die bedruckten Seiten der Folien 253 und 254 in Kontakt und bilden elektrische Brücken abhängig vom ausgeübten Druck. Dabei ändert sich der messbare elektrische Widerstand und die Grösse des ausgeübten Drucks lässt anhand desselben bestimmen.

Aussenseitig, d. h. auf der der ersten Folie 253 abgewandten Seite, der Folie 254 ist ein ultraschalldurchlässiges Druckübertragungselement 261 vorgesehen. Das Druckübertragungselement 261 weist in diesem Ausführungsbeispiel eine Höhenerstreckung H auf, die zumindest dem Abstand A der Folien 253 und 254 des Foliendrucksensors 251 entspricht. Das Druckübertragungselement 261 weist in diesem Ausführungsbeispiel eine kreisrunde Ausdehnung mit einem Durchmesser d auf, die etwa 40% bis 60% der entsprechenden Ausdehnung D des Zwischenraums 256 des Foliendrucksensors 251 entspricht.

Weiter ist eine Halteeinrichtung 281 vorgesehen, mittels der die Druckmessvorrichtung 251 auf einem Gewebe temporär festlegbar ist. Die Halteeinrichtung umfasst über die Ausdehnung des Reservoirs 271 hinausragende Halteabschnitte 282, welche einseitig mit einer Klebeschicht 283 versehen sind. Als Kleber für die Kleberschicht 283 kommt vorteilhaft ein Kleber zur Anwendung, wie er auch bei Pflastern verwendet wird.

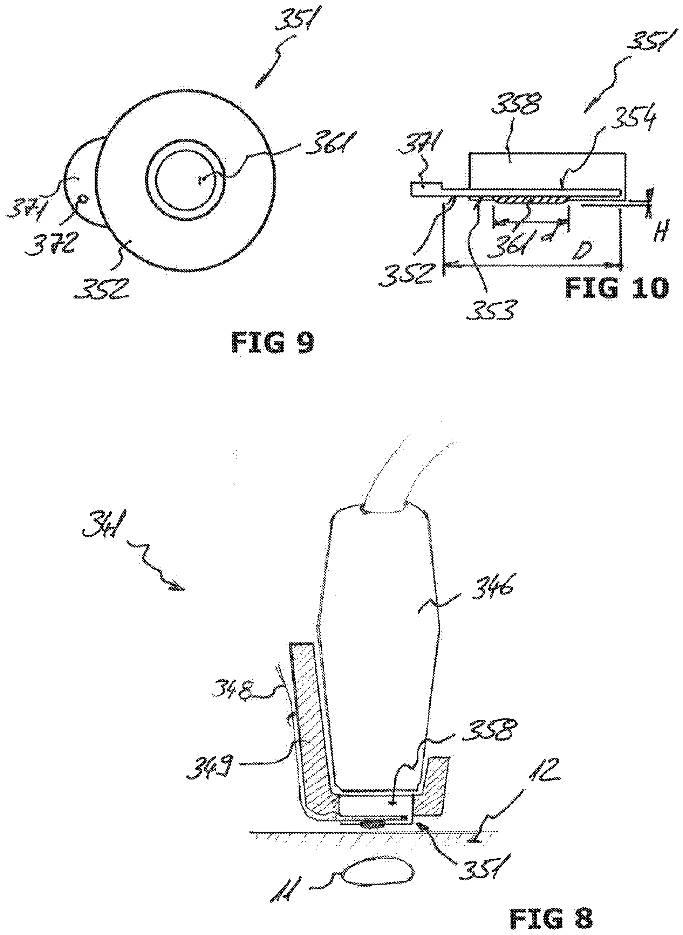

Die in den Figuren 9 und 10 dargestellte Druckmessvorrichtung 351 weist ebenfalls einen Foliendrucksensor 352 auf, der im Aufbau im Wesentlichen den vorgenannten Foliendrucksensoren 52, 102 oder 252 entspricht. Die im Zusammenhang mit diesen Foliendrucksensoren 52, 102 oder 252 gemachten Ausführungen gelten analog.

Der Foliendrucksensor 352 ist in einem ultraschalldurchlässigen Einbettungsmaterial 358 eingebettet. Das Einbettungsmaterial 358 ist ein ultraschalldurchlässiges Silikon, welches bereits im Handel erhältlich ist. Beispielsweise wird das Silikon mit dem Produktnamen Köraform A 42 der Firma Bezema verwendet. Das Silikon lässt sich in beliebige Formen vergiessen.

Der Foliensensor 352 ist - wie in den Figuren 8 bis 10 gezeigt - vollständig in dem Einbettungsmaterial 358 eingebettet.

In einer alternativen, in den Figuren nicht gezeigten Ausführungsform kann der Foliensensor 352 auch nur bereichsweise in dem Einbettungsmaterial 358 eingebettet sein. In einer weiteren alternativen, in den Figuren nicht gezeigten Ausführungsform kann der Foliensensor 352 auch auf einen Block aus einem ultraschalldurchlässigen Material angeordnet sein, beispielsweise aufgeklebt.

Der Einbettungsmaterial-Guss ist bevorzugt rund beziehungsweise der Ausgestaltung des verwendeten Foliendrucksensors angepasst. Die Abmessungen des Einbettungsmaterials werden so gewählt, dass ein für die Verwendung genügend grosses Ultraschall Fenster entsteht, das es noch erlaubt, die auszumessenden Strukturen im Ultraschallbild abzubilden.

Die Stärke des Einbettungsmaterials 358 wird vorteilhaft dünn ausgebildet, damit der Einfluss desselben auf die Ultraschallmessung so gering wie möglich ist. In den durchgeführten Versuchen hat sich eine Dicke in einem Bereich von 2 mm bis 15 mm, bevorzugt in einem Bereich von 3 mm bis 10 mm, als besonders vorteilhaft erwiesen.

Ein Druckübertragungselement 361 ist an der mit der Haut bzw. dem Gewebe 12 in Anlage bringbare Aussenseite des Foliendrucksensors 352 beziehungsweise der Druckmessvorrichtung 351 vorgesehen. Das Druckübertragungselement 361 dient der Kraftübertragung von der flexiblen Haut bzw. dem Gewebe 12 auf den Foliendrucksensor 352. Vorteilhaft ist das Druckübertragungselement 361 im Zentrum des Foliendrucksensors 352 angeordnet.

Das Druckübertragungselement 361 weist eine Ausdehnung d auf, die etwa 30% bis 70% der entsprechenden Ausdehnung D des Zwischenraums 356 des Foliendrucksensors 352 entspricht. Das Druckübertragungselement 361 weist weiter eine Höhenerstreckung H auf, die etwa dem 5-fachen bis 20-fachen des Abstands der Folien 353, 354 des Foliendrucksensors 352 zueinander entspricht.

Die Ausdehnung D des Zwischenraums 356 des Foliendrucksensors 352 ist kleiner als die elektrisch-aktive Messfläche des Foliendrucksensors 352.

Beispielsweise wird als Foliendrucksensor 352 ein Force Sensing Resistor (FSR) der Firma Interlinks Electronics, z. B. des Typs FSR 402 long, verwendet, der eine aktive Messfläche von 22 mm2 aufweist. Bei einer Verwendung dieses Typs von Foliendrucksensor 352 hat sich in Versuchen eine Höhenerstreckung im Bereich von 0.5 mm bis 3 mm als vorteilhaft erwiesen.

Weiter ist ein Reservoir 371 für die ultraschalltransparente sowie nicht-elektrolytisch aktive Flüssigkeit vorgesehen. Zwischen dem Reservoir 371 und dem Zwischenraum 356 des Foliendrucksensors 352 ist eine Flüssigkeitsverbindung, z. B. in Form eines Kanals oder eines Leitungsabschnitts, vorgesehen. Mit der Flüssigkeitsverbindung wird sichergestellt, dass bei der Verwendung der Druckmessvorrichtung 351 die ultraschalltransparente sowie nicht-elektrolytisch aktive Flüssigkeit aus dem Zwischenraum 356 im Foliendrucksensors 352 austreten und wieder eintreten kann, ohne dass eine störende Luftvermischung auftritt, welche die Ultraschall-Leitfähigkeit der Flüssigkeit und damit die Bildgebung beeinträchtigen würde.

Vorteilhaft ist eine vorteilhaft verschliessbare Befüllöffnung 372 vorgesehen, über welche bedarfsweise die ultraschalltransparente sowie nicht-elektrolytisch aktive Flüssigkeit zugeführt oder abgeführt werden kann.

Bei dem in Fig. 8 dargestellten Druckmesssystem 341 zur Druckmessung einer Vene 11 oder eines Organs die Druckmessvorrichtung 351 und die Ultraschallmesseinheit 346 zu einer Messeinheit verbunden.

Zur Kopplung der Druckmessvorrichtung 351 mit der Ultraschallmesseinheit 346 ist ein Adapter 349 vorgesehen, der in diesem Ausführungsbeispiel L-förmig ausgebildet ist. Weitere mögliche Ausgestaltungen des Adapters stellen - nicht abschliessend - flache oder U-förmige Ausbildungen dar.

Der Adapter 349 dient als Verbindungsstück zum Ultraschallkopf 346. Der Adapter 349 sichert die Position der Druckmessvorrichtung 351 vor dem Ultraschallkopf 346, damit der auszuübende Druck auf die Druckmessvorrichtung 351 übertragen werden kann.

Der Adapter 349 kann aus einem Einbettungsmaterial 358, beispielsweise aus einem Silikon, hergestellt sein. Vorteilhaft wird dabei das gleiche Einbettungsmaterial 358 verwendet, welches zur Einbettung des Foliensensors 352 vorgesehen ist. Damit lassen sich gegebenenfalls mehrere Arbeitsschritte bei der Herstellung des Druckmesssystems 341 einsparen.

Eine Datenleitung 348 ist zur Weitergabe der vom Foliendrucksensor 352 ermittelten Werte vorgesehen.

In den Lücken zwischen dem Ultraschallkopf 346 und dem Einbettungsmaterial 358 sowie zwischen der Druckmessvorrichtung 351 und der Haut bzw. dem Gewebe 12 ist vorteilhaft ein ultraschalldurchlässiges Gel oder eine ultraschalldurchlässige Flüssigkeit vorzusehen, z. B. ein kommerziell und für die Anwendung zugelassenes Ultraschall Gel.

Insbesondere lassen sich mit den erfindungsgemässen Druckmessvorrichtungen 51, 101, 151, 251 bzw. 351 Venendrücke und Organdrücke bestimmen und Elastizitätsmessungen von Organen und Geweben insbesondere von Kompartimenten durchführen. Dies erlaubt bspw. eine schnelle und kostengünstige, nicht-invasive Messung des peripheren und zentralen Venendruckes (ZVD). Weiter ermöglicht dies eine kostengünstige, nicht-invasive Messung der Elastizität in einer Muskelloge zur Beurteilung des Druckes innerhalb der Loge (sogenanntes Logensyndrom). Die bisherige konventionelle Messung des ZVD erfolgt invasiv durch Einführen eines Katheters in die obere Hohlvene. Eine solche Messung hat eine Komplikationsrate von ungefähr 20% und ist mit teilweise gravierenden Risiken für Patienten verbunden. Zudem dauert die Einlage des Katheters ca. 23 Minuten und muss von zwei Personen, von denen zwingend eine ein Arzt muss, vorgenommen werden. Der Eingriff ist relativ kostspielig. Demgegenüber stellt das erfindungsgemässe Druckmesssystem 41, 141 bzw. 341 eine nicht-invasive schnellere, einfachere, günstigere und komplikationsfreie Messmethode zur Venendruckmessung zur Verfügung. Die Druckmessung und/oder Elastizitätsmessung kann hierbei durch geschultes Personal bzw. angelernte Untersucher durchgeführt werden.

Der Einsatz des erfindungsgemässen Druckmesssystems 41, 141 und 341 führt zur schnelleren Betriebsabläufen und erlaubt eine weitestgehend automatisierte Messung des zentralen Venendruckes, ähnlich dem von im Handel üblichen Blutdruckmessgeräten für den arteriellen Blutdruck. Hierzu wird eine Kombination aus einem Ultraschallmesskopf 46, 146 oder 346 und einer erfindungsgemässen Druckmessvorrichtung 51, 101, 151, 251 oder 351 auf zu untersuchendes Patientengewebe beispielsweise am Arm eines Patienten aufgesetzt und über die Druckmessvorrichtung 51, 101, 151, 251 oder 351 wird, wie in den obigen Ausführungsbeispielen dargestellt, der aus der Aufsetzkraft resultierende Druck im Gewebe 12 gemessen. Gleichzeitig wird mittels Ultraschall beobachtet, wie sich eine im Gewebe 12 befindliche Vene 11 verhält. Sobald beispielsweise die Vene 11 kollabiert, kann der zugehörige Druck im Gewebe 12 ermittelt werden, welcher dann dem Druck des Blutes in der Vene 11 entspricht.

Bei einer Elastizitätsmessung einer Vene 11, eines Organs oder eines Kompartimentes wird die aus der Aufsetzkraft resultierende Elastizität im Gewebe 12 gemessen und daraus ein Elastizitätsquotient ermittelt. Der ermittelte Elastizitätsquotient wird dann mit dem Organdruck korreliert.

Selbstverständlich ist die Messmethode nicht auf Druckmessungen und/oder Elastizitätsmessung von Venen beschränkt, sondern kann auch bei anderen Gewebestrukturen, insbesondere bei Organen oder Organteilen (z. B. der Leber oder der Milz, an Muskelgewebe und Muskellogen) sowie Kompartimenten des Körpers und andere Körperflüssigkeiten Anwendung finden. Die vorgenannten Vorrichtungen können zur nicht-invasiven Messung des intrakraniellen Druckes bei Neugeborenen mit noch offener Fontanelle Anwendung finden.

Vorteilhaft kann der Messvorgang automatisch gestartet werden. Sobald ein Druckimpuls vom Foliendrucksensor 52, 102, 152, 252 bzw. 352 gemessen wird, kann zum Beispiel die Verarbeitungseinrichtung 21 eingeschaltet werden, ferner kann ebenso über ein geeignetes Signal die Ultraschallmesseinheit 46, 146 bzw. 346 gestartet werden. Ein automatischer Startvorgang hat den Vorteil, dass das erfindungsgemässe Druckmesssystem 41, 141 bzw. 341 energieeffizient eingesetzt werden kann. Es ist ferner denkbar, dass über geeignete Analysemethoden automatisch der Zeitpunkt festgestellt werden kann, an dem eine Vene 11 oder ein anderes Körperflüssigkeitsgefäss kollabiert. Beispielsweise ist es denkbar, dass über ein Körperschallmikrofon die Veränderung eines Blutströmungsgeräusches die Veränderung im Strömungsgeräusch als Indikator für das Kollabieren einer Vene 11 verwendet wird. Ebenfalls ist es denkbar, über bildverarbeitende Verfahren erzeugte Abbilder vom Inneren des Gewebes 12 in einer Extremität (z. B. in einem Arm) automatisch zu analysieren, um die Lage einer Vene 11 aufzuspüren und deren Geometrieveränderung zu bestimmen. Auf diese Weise kann ebenfalls eine Detektion des Kollabierens der Vene 11 erfolgen, um den Zeitpunkt der korrekten Messung des Venendruckes festzulegen. Dieser Zeitpunkt wird dann im zeitlichen Verlauf der Druckmessung als Zeitpunkt festgelegt an dem der zentrale Venendruck ermittelt wurde.

Ferner kann vorteilhaft auf einer Anzeigeeinheit 23 gleichzeitig zum Druckverlauf ein Abbild der mittels Ultraschall beobachteten Vene 11 nebeneinander oder übereinander dargestellt werden. Eine derartige Ausführungsform bietet den Vorteil einer kompakten Darstellung und erlaubt es lediglich, eine Anzeigevorrichtung 23 für mehrere Anzeigefunktionen bereitstellen zu müssen.

Bei weiteren Ausführungsformen ist es beispielsweise denkbar, die von der erfindungsgemässen Druckmesseinrichtung gemessenen Messwerte zu sammeln und zur Qualitätskontrolle zu verwenden, um insbesondere die richtige Position der Druckermittlung, das heisst die korrekte Lage über einer Vene 11, nachvollziehen zu können und nachvollziehen zu können, ob die richtige und eine ausreichende Kraft für die Druckmessung bzw. die Elastizitätsmessung auf das Gewebe 12 ausgeübt wurde.

Generell haben automatisierte Verfahren in Ausführungsformen der erfindungsgemässen Druckmessvorrichtung 16 und des erfindungsgemässen Druckmesssystems 41, 141 bzw. 341 den Vorteil, dass weniger Fehler auftreten. Ferner ist es auch denkbar, das Verfahren zur Messung der Lebersteifigkeit einzusetzen. Hierzu wird die Leber mittels des zunehmenden Drucks in der Messflüssigkeit bis zu einem bestimmten Verformungsgrad deformiert, wobei das Verhältnis von Verformungsgrad zum Druck das Mass für die Lebersteifigkeit darstellt. Alternativ kann die Lebersteifigkeit auch gemessen werden indem der Druck einer Lebervene ermittelt und im Umkehrschluss wird festgestellt, dass, je höher der Druck in der Lebervene ist, desto steifer das Lebergewebe ist.

Im Folgenden werden mögliche Benutzerfehler bei der Venendruckmessung und Massnahmen dagegen beschrieben.
- Die Anordnung der Druckmessvorrichtung ist zu weit von der Achse in Bezug auf die Zielvene entfernt und deswegen wird kein Spitzendruck auf die Vene ausgeübt. Daher sollte bevorzugt das erfindungsgemässe Verfahren zur Venendruckmessung eine Detektion der Mittelachse des Druckmesssystems und einen Vergleich derselben mit der Zielvene durchführen. Bevorzugt wird die Zielvene dabei farblich auf der Anzeigeeinheit markiert. Befindet sich die Druckmessvorrichtung nicht in einer idealen Position für eine genaue Druckmessung, erfolgt vorzugsweise eine Warnung des Untersuchers mit Aufforderung zur Korrektur der Position der Druckmessvorrichtung und es erfolgt keine Freigabe der Druckmessung.
- Die Ausübung des Drucks durch die Druckmessvorrichtung auf die Vene erfolgt ungleichmässig und führt zur asymmetrischen Gewebeverschiebung zwischen der Druckmessvorrichtung, die das Gewebe 12 und die Vene 11 berührt. Daher werden erfindungsgemäss bevorzugt Oberflächen-Verschiebungsverfahren und elastographische Verfahren angewendet werden, um solche Fälle aufzuspüren. Vorzugsweise erfolgt in einem solchen Fall eine Warnung des Untersuchers mit Aufforderung zur Korrektur mit Aufforderung zur gleichmässigen Druckaufbringung und es erfolgt keine Freigabe der Druckmessung.
- Eine Anordnung der Druckmessvorrichtung erfolgt an einen ungeeigneten Ort. Als Abhilfe ist erfindungsgemäss eine Auswertung des Kontaktbereiches für die Druckmessvorrichtung vorgesehen. Beispielsweise wird ein geeigneter Ort zur Venendruckmessung durch Suchen nach innenliegendem Knochen unterhalb der Zielvene 11 gefunden. Vorzugsweise erfolgt eine Warnung des Untersuchers mit Aufforderung zur Korrektur der Platzierung der Druckmessvorrichtung und es erfolgt keine Freigabe der Druckmessung.
- Auch ist vorteilhaft eine sichtbare Darstellung des Messzyklus vorgesehen. Daran kann ein automatisches Aufspüren des Zeitpunktes, an dem die Messung begonnen wurde, durch Auswertung der Venengeometrie und ihrer Verformung erfolgen (Beispielsweise Beginn der Messung bei 5% Geometrieveränderung von Venenhöhe zu Venenbreite).
   Ferner kann bevorzugt eine Benachrichtigung des Untersuchers über die Tatsache erfolgen, dass die Messung begonnen hat. Zum Beispiel kann dies durch Wechseln der Farben von bestimmten Bereichen des Bildschirms bzw. der Anzeigeeinheit 23 geschehen. Vorteilhaft wird so sichergestellt, dass der Untersucher weiss, dass ein Messzyklus begonnen wurde, und ferner wird dem Untersucher ein weiteres Datum zur Auswertung in Form des Ausgangsdrucks bei der ersten Bewegung der Venengeometrie zur Verfügung gestellt.
- Auch ist eine sichtbare Darstellung des Endes des Messzyklus vorgesehen. Dies gestattet ein automatisches Aufspüren des Zeitpunktes, an dem die Vene 11 kollabiert ist und die Messung vervollständigt wurde, durch Auswertung der Venengeometrie und ihrer Verformung. Zum Beispiel kann "Beenden" vorzugsweise definiert sein, wenn 95% der Geometrieveränderung von Venenhöhe zu Venenbreite vorliegen, oder sobald kein hohler Bereich in der Vene 11 mehr detektiert werden kann.
   Bevorzugt erfolgt eine Benachrichtigung des Untersuchers über die Tatsache, dass die Messung abgeschlossen wurde. Dies geschieht zum Beispiel durch Wechseln der Farben von bestimmten Bereichen auf dem Bildschirm und durch Anzeige des Messergebnisses in, vorteilhaft grossen, Ziffern auf der Anzeigeeinheit (Bildschirm). Vorteilhaft wird so sichergestellt, dass der Untersucher weiss, dass ein Messzyklus beendet ist. Diese Daten können verwendet werden und ebenso kann eine neue Messung begonnen werden.
- Vorzugsweise kann erfindungsgemäss sichergestellt werden, dass die Fortführung der Messung durch weitere Datensammlung an derselben Position erfolgt. Daher erfolgt bevorzugt eine automatische Detektion von weiteren Messungen, die es gestattet, dem Untersucher nach einer schlechten Messung eine weitere Messung unter Berücksichtigung der oben erwähnten Kriterien durchzuführen. Falls der Messzyklus abgeschlossen ist, wird die neue Messung ebenso angezeigt. Bevorzugt wird ein Mittelwert mit den vorangehenden Messungen am gleichen Ort ebenso angezeigt mit dem Wert der vorangehenden Messung.
   Ein fortwährendes Überwachen des Drucks und des analysierten Bildes resultieren vorteilhaft in einer automatischen Detektion des Messendes. Sobald zum Beispiel der Untersucher die Druckmessvorrichtung von der Haut des Patienten abhebt oder sie zu weit vom Ausgangsort der Messung entfernt bzw. verschiebt, kann vorteilhaft die Messung beendet werden. Fallweise kann danach vorteilhaft ein neuer Messzyklus angestossen werden, sobald der Untersucher den Betätigungsknopf an der Druckmessvorrichtung drückt oder die Druckmessvorrichtung mit der Haut in Berührung kommt und der Druck ansteigt, wobei vorteilhaft Gewebe 12 durch eine Auswertungssoftware detektiert werden kann. Eine solche Option kann beispielsweise von einem persönlichen Einstellmenü innerhalb der Software ausgewählt werden.
- Um den Untersucher nicht zu verwirren und zum Zwecke einer übersichtlichen Darstellung, werden vorteilhaft die gemessenen und ausgewerteten Daten in einem kombinierten Rahmen zusammen mit dem Ultraschallbild angezeigt, das durch die medizinische Ultraschallvorrichtung abgegeben wird.
- Um einem Untersucher aktuelle Informationen über die Qualität des Messzyklus zu geben, sollten die detektierten Typologien von Haut, Oberfläche der Venendruckvorrichtung, Vene, Knochen; wie oben dargelegt, auf dem Bildschirm angezeigt werden und farblich gemäss ihrem Status eingefärbt werden. Beispielsweise bedeutet Topologie grün "Kein Problem", rot "nicht akzeptabel" und orange "kritisch".

Das erfindungsgemässe Verfahren zur Druckmessung und/oder Elastizitätsmessung einer Vene oder eines Organs gibt eine einfache Vorgehensweise an, womit eine aus Druckmessvorrichtung und Ultraschallmesseinheit bestehende Druckmessvorrichtung zum automatisierten Messen von Venen- und oder Organdrücken bzw. von Venen- und oder Organelastizitäten eingesetzt werden kann.

Für den Fachmann sind angesichts der voranstehenden Beschreibung und Ausführungsbeispielen der erfindungsgemässen Druckmessvorrichtung, des Druckmesssystems und des Verfahrens Druckmessung und/oder Elastizitätsmessung einer Vene oder eines Organs Abwandlungen im Rahmen der technischen Möglichkeiten und der bekannten Vorrichtungen zur Druck- und Ultraschallmessungen denkbar.

### Bezugszeichenliste

- 11: Vene
- 12: Gewebe

- 14: Kraft (Pfeil)

- 16: Messeinrichtung

- 21: Verarbeitungseinheit
- 22: Recheneinheit
- 23: Anzeigeeinheit

- 41: Druckmesssystem

- 46: Ultraschallmesseinheit
- 47: Datenleitung

- 51: Druckmessvorrichtung
- 52: Foliendrucksensor
- 53: (untere) Folie
- 54: (obere) Folie

- 56: Zwischenraum

- 58: Datenleitung

- 61: Druckübertragungselement

- 71: Reservoir
- 101: Druckmessvorrichtung
- 102: Foliendrucksensor

- 121: Reservoir

- 141: Druckmesssystem

- 146: Ultraschallmesseinheit
- 147: Sender

- 151: Druckmessvorrichtung

- 158: Datenleitung

- 251: Druckmessvorrichtung
- 252: Foliendrucksensor
- 253: (untere) Folie
- 254: (obere) Folie
- 255: Abstandhalter
- 256: Zwischenraum

- 261: Druckübertragungselement

- 271: Reservoir

- 281: Halteeinrichtung
- 282: Halteabschnitt
- 283: Kleberschicht
- H: Höhenerstreckung v. 261 bzw. 361
- A: Abstand zw. Folien (253/254)
- d: Durchmesser v. 261 bzw. 361
- D: Ausdehnung v. 256 bzw. 356

- 341: Druckmesssystem

- 346: Ultraschallmesseinheit

- 348: Datenleitung
- 349: Adapter

- 351: Druckmessvorrichtung
- 352: Foliendrucksensor
- 353: (untere) Folie
- 354: (obere) Folie

- 358: Einbettungsmaterial

- 361: Druckübertragungselement

- 371: Reservoir
- 372: Befüllöffnung

## Patentansprüche

1. Druckmessvorrichtung zur Druckmessung und/oder Elastizitätsmessung einer Vene (11), eines Organs oder eines Kompartimentes und zur Kombination mit einer Ultraschallmesseinheit (46; 146; 346), **dadurch gekennzeichnet, dass** ein als Foliendrucksensor (52; 102; 252; 352) ausgebildeter Drucksensor vorgesehen ist, wobei ein Zwischenraum (56; 256; 356) zwischen den Folien (53, 54; 253, 254) des Foliendrucksensors (52; 102; 252; 352) mit einer ultraschalltransparenten sowie nicht-elektrolytisch aktiven Flüssigkeit befüllt ist.

2. Druckmessvorrichtung nach Anspruch 1, **dadurch gekennzeichnet, dass** die Folien (53, 54; 253, 254) des Foliendrucksensors (52; 102; 252; 352) zumindest bereichsweise aus einem ultraschalldurchlässigen Material gefertigt sind.

3. Druckmessvorrichtung nach Anspruch 1 oder 2, **dadurch gekennzeichnet, dass** die ultraschalltransparente sowie nicht-elektrolytisch aktive Flüssigkeit eine Flüssigkeit aus der Gruppe der wässrigen Flüssigkeiten, Ultraschall-durchlässige Gele, synthetische Öle oder biologische Öle ist.

4. Druckmessvorrichtung nach einem der vorangehenden Ansprüche, **dadurch gekennzeichnet, dass** zumindest ein Druckübertragungselement (61; 261; 361) vorgesehen ist.

5. Druckmessvorrichtung nach Anspruch 4, **dadurch gekennzeichnet, dass,** das zumindest eine Druckübertragungselement (61; 261) an einer mit der Ultraschallmesseinheit (46; 146) in Anlage bringbare Aussenseite des Foliendrucksensors (52; 252) oder der Druckmessvorrichtung (51; 101; 151; 251) angeordnet ist, wobei das Druckübertragungselement (61; 261) vorteilhaft eine Höhenerstreckung (H) aufweist, die zumindest dem Abstand (A) der Folien (253, 254), vorteilhaft dem 5-fachen bis 20-fachen des Abstands (A) der Folien (253, 254), des Foliendrucksensors (252) zueinander entspricht, und weiter vorteilhaft eine Ausdehnung (d) aufweist, die etwa 30% bis 70%, insbesondere 40% bis 60%, der entsprechenden Ausdehnung (D) des Zwischenraums (256) des Foliendrucksensors (252) entspricht.

6. Druckmessvorrichtung nach Anspruch 4 oder 5, **dadurch gekennzeichnet, dass,** das zumindest eine Druckübertragungselement (361) an einer mit der Haut bzw. dem Gewebe (12) in Anlage bringbare Aussenseite des Foliendrucksensors (352) oder der Druckmessvorrichtung (351) angeordnet ist, wobei das zumindest eine Druckübertragungselement (361) vorteilhaft eine Ausdehnung (d) aufweist, die etwa 30% bis 70% der entsprechenden Ausdehnung (356) des Foliendrucksensors (352) entspricht und weiter vorteilhaft eine Höhenerstreckung (H) aufweist, die etwa 5% bis 20% der entsprechenden Ausdehnung (D) des Zwischenraums (356) des Foliendrucksensors (352) entspricht.

7. Druckmessvorrichtung nach einem der vorangehenden Ansprüche, **dadurch gekennzeichnet, dass** ein Reservoir (71; 121; 271; 371) für die ultraschalltransparente sowie nicht-elektrolytisch aktive Flüssigkeit vorgesehen ist, wobei zwischen dem Reservoir (71; 121; 271; 371) und dem Zwischenraum (56; 256; 356) des Foliendrucksensors (52; 102; 252; 352) eine Flüssigkeitsverbindung vorgesehen ist, wobei vorteilhaft der Foliendrucksensor (52; 102; 252; 352) in dem Reservoir (71; 121; 271; 371) angeordnet ist.

8. Druckmessvorrichtung nach einem der vorangehenden Ansprüche, **dadurch gekennzeichnet, dass** eine Datenleitung (58; 158; 358) zur Weitergabe der vom Foliendrucksensor (52; 102; 252; 352) ermittelten Werte vorgesehen ist oder dass ein Sender (147) zur Weitergabe der vom Foliendrucksensor (52; 102; 252) ermittelten Werte vorgesehen ist.

9. Druckmessvorrichtung nach einem der vorangehenden Ansprüche, **dadurch gekennzeichnet, dass** eine Halteeinrichtung (281) für die Druckmessvorrichtung (251) vorgesehen ist, welche vorteilhaft auf einem Gewebe temporär festlegbar ist.

10. Druckmessvorrichtung nach einem der vorangehenden Ansprüche, **dadurch gekennzeichnet, dass** der Foliendrucksensor (352) zumindest bereichsweise in einem ultraschalldurchlässigen Einbettungsmaterial, vorzugsweise einem ultraschalldurchlässigen Silikon, eingebettet ist, wobei die Einbettung des Foliendrucksensors (352) vorteilhaft zumindest an der mit der Haut bzw. Gewebe (12) in Anlage bringbare Aussenseite des Foliendrucksensors (352) vorgesehen ist.

11. Druckmesssystem zur Druckmessung und/oder Elastizitätsmessung einer Vene (11) oder eines Organs wenigstens aufweisend eine Druckmessvorrichtung (51; 101; 151; 251) nach einem der Ansprüche 1 bis 10 und eine Ultraschallmesseinheit (46; 146; 346), wobei die Druckmessvorrichtung (51; 101; 151; 251; 351) und die Ultraschallmesseinheit (46; 146; 346) miteinander, vorteilhaft mechanisch, zu einer Messeinheit gekoppelt sind oder zu einer Messeinheit verbunden sind.

12. Messeinrichtung zur Druckmessung und/oder Elastizitätsmessung einer Vene (11), eines Organs oder eines Kompartimentes wenigstens aufweisend eine Verarbeitungseinheit (21) und ein Druckmesssystem, das eine Ultraschallmesseinheit (46; 146; 346) und eine Druckmessvorrichtung (51; 101; 151; 251, 351) nach einem der Ansprüche 1 bis 10 aufweist.

13. Verfahren zur Druckmessung und/oder Elastizitätsmessung einer Vene (11), eines Organs oder eines Kompartimentes, bei dem eine mit einer Druckmessvorrichtung (51; 101; 151; 251; 351) nach einem der Ansprüche 1 bis 10 gekoppelte Ultraschallmesseinheit (46; 146; 346) oder bei dem ein Drucksystem (41; 141) nach Anspruch 11 auf ein Gewebe (12), unter dem sich die zu messende Vene (11) bzw. das zu messende Organ oder Kompartiment befindet, aufgesetzt wird und solange der Druck auf das Gewebe (12) gesteigert wird, bis sich aus der Ultraschallmessung ergibt, dass die Vene (11) kollabiert bzw. das Organ oder das Kompartiment einen vorbestimmten Verformungsgrad erreicht, wobei der zu diesem Zeitpunkt anliegende Druck bzw. die zu diesem Zeitpunkt vorhandene Elastizität über die Druckmessvorrichtung (51; 101; 151; 251; 351) als Venendruck bzw. Organdruck oder Kompartimentdruck bestimmt wird, wobei die Messinformationen über Funk oder eine Datenleitung (47, 58; 158; 358) an eine Verarbeitungseinheit (21) weitergeleitet werden.

14. Verfahren nach Anspruch 13, **dadurch gekennzeichnet, dass** der Zeitpunkt der Druckmessung bzw. der Elastizitätsmessung in der Verarbeitungseinheit (21) automatisch ermittelt wird und/der der Zeitpunkt des Kollabierens einer Vene (11) oder das Erreichen eines vorbestimmten Verformungsgrades eines Organs oder eines Kompartimentes über ein bildverarbeitendes Verfahren bestimmt wird.

## Claims

1. A pressure measurement device for measuring pressure and/or for measuring elasticity of a vein (11), an organ or a compartment and for combination with an ultrasound measurement unit (46; 146; 346), **characterized in that** a pressure sensor, configured as a film pressure sensor (52; 102; 252; 352) is provided, wherein an intermediate space (56; 256; 356) between the films (53, 54; 253, 254) of the film pressure sensor (52; 102; 252; 352) is filled with an ultrasound-transparent and non-electrolytically active liquid.

2. The pressure measurement device according to Claim 1, **characterized in that** the films (53, 54; 253, 254) of the film pressure sensor (52; 102; 252; 352) are manufactured from an ultrasound-transparent material, at least in some regions.

3. The pressure measurement device according to Claim 1 or 2, **characterized in that** the ultrasound-transparent and non-electrolytically active liquid is a liquid selected from the group of aqueous liquids, ultrasound-transparent gels, synthetic oils or biological oils.

4. The pressure measurement device according to one of the preceding claims, **characterized in that** at least one pressure transmission element (61; 261; 361) is provided.

5. The pressure measurement device according to Claim 4, **characterized in that** at least one pressure transmission element (61; 261) is arranged on an outer side of the film pressure sensor (52; 252) which can be brought to abut with the ultrasound measurement unit (46; 146), or the pressure measurement device (51; 101; 151; 251), wherein the pressure transmission element (61; 261) advantageously has a height extension (H) which at least corresponds to the spacing (A) of the films (253, 254), advantageously 5 to 20 times the spacing (A) of the films (253, 254) of the film pressure sensor (252) to one another, and further advantageously has an extension (d) which corresponds to approximately 30% to 70%, in particular 40% to 60%, of the corresponding extension (D) of the intermediate space (256) of the film pressure sensor (252) .

6. The pressure measurement device according to Claim 4 or 5, **characterized in that** the at least one pressure transmission element (361) is arranged on an outer side of the film pressure sensor (352), or the pressure measurement device (351), which can be brought to abut with the skin or the tissue (12), wherein the at least one pressure transmission element (361) advantageously has an extension (d) which corresponds to approximately 30% to 70% of the corresponding extension (356) of the film pressure sensor (352), and further advantageously has a height extension (H) which corresponds to approximately 5% to 20% of the corresponding extension (D) of the intermediate space (356) of the film pressure sensor (352) .

7. The pressure measurement device according to one of the preceding claims, **characterized in that** a reservoir (71; 121; 271; 371) is provided for the ultrasound-transparent and non-electrolytically active liquid, wherein between the reservoir (71; 121; 271; 371) and the intermediate space (56; 256; 356) of the film pressure sensor (52; 102; 252; 352), a liquid connection is provided, wherein advantageously the film pressure sensor (52; 102; 252; 352) is arranged in the reservoir (71; 121; 271; 371).

8. The pressure measurement device according to one of the preceding claims, **characterized in that** a data line (58; 158; 358) is provided for relaying the values determined by the film pressure sensor (52; 102; 252; 352), or that a transmitter (147) is provided for relaying the values determined by the film pressure sensor (52; 102; 252).

9. The pressure measurement device according to one of the preceding claims, **characterized in that** a holding device (281) is provided for the pressure measurement device (251), which can be advantageously fixed temporarily on a tissue.

10. The pressure measurement device according to one of the preceding claims, **characterized in that** the film pressure sensor (352), at least in some regions, is embedded in an ultrasound-transparent embedding material, preferably an ultrasound-transparent silicone, wherein the embedding of the film pressure sensor (352) is provided advantageously at least on the outer side of the film pressure sensor (352) which can be brought to abut with the skin or tissue (12).

11. A pressure measurement system for measuring pressure and/or for measuring elasticity of a vein (11) or an organ, at least comprising a pressure measurement device (51; 101; 151; 251) according to one of Claims 1 to 10, and an ultrasound measurement unit (46; 146; 346), wherein the pressure measurement device (51; 101; 151; 251; 351) and the ultrasound measurement unit (46; 146; 346) are coupled to one another, advantageously mechanically, to a measurement unit or are connected to a measurement unit.

12. A measurement device for measuring pressure and/or for measuring elasticity of a vein (11), an organ or a compartment, at least comprising a processing unit (21) and a pressure measurement system, which comprises an ultrasound measurement unit (46; 146; 346) and a pressure measurement device (51; 101; 151; 251; 351) according to one of Claims 1 to 10.

13. A method for measuring pressure and/or for measuring elasticity of a vein (11), an organ or a compartment, in which an ultrasound measurement unit (46; 146; 346) coupled to a pressure measurement device (51; 101; 151; 251; 351) according to one of Claims 1 to 10, or in which a pressure system (41; 141) according to Claim 11 is placed on a tissue (12), below which the vein (11) to be measured or the organ or compartment to be measured in located, and the pressure on the tissue (12) is increased until the ultrasound measurement reveals that the vein (11) collapses or the organ or the compartment reaches a predetermined degree of deformation, wherein the pressure applied at this point in time, or the elasticity present at this point in time is determined by means of the pressure measurement device (51; 101; 151; 251; 351) as venous pressure or organ pressure or compartment pressure, wherein the measurement information is relayed via radio or a data line (47, 58; 158; 358) to a processing unit (21).

14. The method according to Claim, 13, **characterized in that** the point in time of the pressure measurement or the elasticity measurement is determined automatically in the processing unit (21) and/or the point in time of the collapse of a vein (11) or the reaching of a predetermined degree of deformation of an organ or a compartment is determined by an image-processing method.

## Revendications

1. Dispositif de mesure de tension pour la mesure de tension et/ou la mesure d'élasticité d'une veine (11), d'un organe ou d'un compartiment et destiné à être combiné avec une unité de mesure échographique (46 ; 146 ; 346), **caractérisé en ce qu'**un capteur de tension réalisé sous forme d'un capteur de tension à membranes (52 ; 102 ; 252 ; 352) est prévu, un espacement (56 ; 256 ; 356) entre les membranes (53, 54 ; 253, 254) du capteur de tension à membranes (52 ; 102 ; 252 ; 352) étant rempli d'un liquide transparent aux ultrasons et non actif du point de vue électrolytique.

2. Dispositif de mesure de tension selon la revendication 1, **caractérisé en ce que** les membranes (53, 54 ; 253, 234) du capteur de tension à membranes (52 ; 102 ; 252 ; 352) sont fabriquées du moins par endroits à partir d'un matériau perméable aux ultrasons.

3. Dispositif de mesure de tension selon la revendication 1 ou 2, **caractérisé en ce que** le liquide actif transparent aux ultrasons et non actif du point de vue électrolytique est un liquide du groupe composé des liquides aqueux, des gels perméables aux ultrasons, des huiles synthétiques ou des huiles biologiques.

4. Dispositif de mesure de tension selon une des revendications précédentes, **caractérisé en ce qu'**il est prévu au moins un élément de transfert de tension (61 ; 261 ; 361).

5. Dispositif de mesure de tension selon la revendication 4, **caractérisé en ce qu'**au moins un élément de transfert de tension (61; 261) est disposé sur une face extérieure pouvant être mise en contact avec l'unité de mesure échographique (46 ; 146) du capteur de tension à membranes (52 ; 252) ou du dispositif de mesure de tension (51 ; 101 ; 151 ; 251), l'élément transfert de tension (61; 261) présentant avantageusement une extension en hauteur (H) qui représente au moins la distance (A) entre les membranes (253, 254), avantageusement 5 fois à 20 fois la distance (A) entre les membranes (253, 254) du capteur de tension à membranes (252), et plus avantageusement présente une extension (d) qui représente environ 30 % à 70 %, en particulier 40 % à 60 %, de l'extension correspondante (D) de l'espacement (256) du capteur de tension à membranes (252).

6. Dispositif de mesure de tension selon la revendication 4 ou 5, **caractérisé en ce qu'**au moins un élément de transfert de tension (361) est disposé sur une face extérieure pouvant être amenée en contact avec la pellicule ou la toile (12) du capteur de tension à membranes (352) ou du dispositif de mesure de tension (351), l'au moins un élément de transfert de tension (361) présentant avantageusement une extension (d) qui représente environ 30 % à 70 % de l'extension correspondante (356) du capteur de tension à membranes (352) et plus avantageusement une extension en hauteur (H) qui représente environ 5 % à 20 % de l'extension correspondante (D) de l'espacement (356) du capteur de tension à membranes (352).

7. Dispositif de mesure de tension selon une des revendications précédentes, **caractérisé en ce qu'**un réservoir (71 ; 121 ; 271 ; 371) est prévu pour le liquide transparent aux ultrasons et non actif du point de vue électrolytique, un raccordement de liquide étant prévu entre le réservoir (71 ; 121 ; 271 ; 371) et l'espacement (56 ; 256 ; 356) du capteur de tension à membranes (52 ; 102 ; 252 ; 352), le capteur de tension à membranes (52 ; 102 ; 252,332) étant avantageusement disposé dans le réservoir (71 ; 121 ; 271 ; 371).

8. Dispositif de mesure de tension selon une des revendications précédentes, **caractérisé en ce qu'**il est prévu une ligne de transmission de données (58 ; 158 ; 358) pour la retransmission des valeurs déterminées par le capteur de tension à membranes (52 ; 102 ; 252 ; 352) ou qu'il est prévu un émetteur (147) pour la retransmission des valeurs déterminées par le capteur de tension à membranes (52 ; 102 ; 252).

9. Dispositif de mesure de tension selon une des revendications précédentes, **caractérisé en ce qu'**il est prévu pour le dispositif de mesure de tension (251) un dispositif de retenue (281) qui peut avantageusement être fixé temporairement sur une toile.

10. Dispositif de mesure de tension selon une des revendications précédentes, **caractérisé en ce que** le capteur de tension à membranes (352) est encastré du moins par endroits dans un matériau d'encastrement perméable aux ultrasons, de préférence un silicone perméable aux ultrasons, l'encastrement du capteur de tension à membranes (352) étant prévu avantageusement au moins sur la face extérieure pouvant être amenée en contact avec la pellicule ou la toile (12) du capteur de tension à membranes (352).

11. Système de mesure de tension pour la mesure de tension et/ou la mesure d'élasticité d'une veine (11) ou d'un organe, présentant au moins un dispositif de mesure de tension (51; 101 ; 151 ; 251) selon une des revendications 1 à 10 et une unité de mesure échographique (46 ; 146 ; 346), le dispositif de mesure de tension (51 ; 101 ; 151 ; 251 ;351) et l'unité de mesure échographique (46 ; 146 ; 346) étant avantageusement couplés entre eux, avantageusement mécaniquement, pour former une unité de mesure ou étant connectés pour former une unité de mesure.

12. Système de mesure de tension pour la mesure de tension et/ou la mesure d'élasticité d'une veine (11), d'un organe ou d'un compartiment, présentant au moins une unité de traitement (21) et un système de mesure de tension qui présente une unité de mesure échographique (46 ; 146 ; 346) et un dispositif de mesure de tension (51; 101 ; 151 ; 251; 351) selon une des revendications 1 à 10.

13. Procédé de mesure de tension et/ou de mesure d'élasticité d'une veine (11), d'un organe ou d'un compartiment, dans lequel une unité de mesure échographique (46 ; 146 ; 346) couplée à un dispositif de mesure de tension (51 ; 101 ; 151 ; 251 ; 351) selon une des revendications 1 à 10 ou dans lequel un système de tension (41 ; 141) selon la revendication 11 est posé sur une toile (12) sous laquelle la veine à mesurer (11) ou l'organe compartiment à mesurer se trouve et tant que la tension augmente sur la toile (12) jusqu'à ce que la mesure échographique indique que la veine (11) s'aplatit ou que l'organe compartiment atteint un degré de déformation prédéfini, la tension appliquée à ce moment ou l'élasticité disponible à ce moment étant définie par le dispositif de mesure de tension (51 ; 101 ; 151 ; 251 ; 351) en tant que tension de veine ou tension d'organe ou tension de compartiment, les informations de mesure étant retransmises par radio ou par une ligne de transmission de données (47, 58 ; 158 ; 358) à une unité de traitement (21).

14. Procédé selon la revendication 13, **caractérisé en ce que** le moment de la mesure de tension ou de la mesure d'élasticité est un automatiquement dans l'unité de traitement (21) et/ou le moment d'aplatissement d'une veine (11) ou l'atteinte d'un degré de déformation prédéfini d'un organe ou d'un compartiment est défini par un procédé de traitement d'images.
